# EUROPEAN PATENT APPLICATION

(11) **EP 4 711 443 A1**
(43) Date of publication of application: **18.03.2026**
(21) Application number: 24929060.2
(22) Date of filing: 21.06.2024
(51) Int. Cl.: C12N 1/20, C12G 3/02, C12C 11/00, C11B 9/00, C12H 6/02, C12P 7/62, A23L 29/00, C12R 1/01

(54) **PRIESTIA SP. BACTERIA AND USE THEREOF**

(30) Priority: 15.03.2024 CN 202410298224
(71) Applicant: Wuliangye Yibin Co., Ltd., Yibin, Sichuan 644007 (CN)
(72) Inventor: ZHAO, Dong, Yibin, Sichuan 644007 (CN); LUO, Qingchun, Yibin, Sichuan 644007 (CN); LU, Yanping, Yibin, Sichuan 644007 (CN); ZHENG, Jia, Yibin, Sichuan 644007 (CN); SU, Jian, Yibin, Sichuan 644007 (CN); LI, Xi, Yibin, Sichuan 644007 (CN); WANG, Xiaomei, Yibin, Sichuan 644007 (CN); ZHANG, Man, Yibin, Sichuan 644007 (CN)
(74) Representative: Potter Clarkson
(86) International application number: PCT/CN2024/100560
(87) International publication number: WO 2025/189602

(57) **Abstract**

The present invention relates to the technical field of winemaking microorganisms, and specifically relates to *Priestia* sp. which can simultaneously produce ethyl 3-hydroxybutyrate, 2,3-dihydrobenzofuran and 4-vinylguaiacol, and the use thereof. In order to solve the problems of there being few types of functional microorganisms that can simultaneously produce ethyl 3-hydroxybutyrate, 2,3-dihydrobenzofuran and 4-vinylguaiacol in existing reports and same needing to be developed, the *Priestia* sp. is provided, which has the deposit number of CGMCC NO. 29032 and can produce ethyl 3-hydroxybutyrate, 2,3-dihydrobenzofuran and 4-vinylguaiacol. Therefore, the *Priestia* sp. has a broad application space in the fields of winemaking, fermented foods and fragrances using ethyl 3-hydroxybutyrate, 2,3-dihydrobenzofuran and 4-vinylguaiacol for flavoring, and also in the field of medical drugs utilizing the antioxidant activity and the anticancer activity of 4-vinylguaiacol.

## Description

### TECHNICAL FIELD

The present disclosure belongs to the technical field of brewing microorganisms, and in particular to *Priestia* sp. capable of simultaneously producing ethyl 3-hydroxybutyrate, 2,3-dihydrobenzofuran and 4-vinylguaiacol, and use thereof.

### BACKGROUND

Chinese Baijiu, renowned for its distinctive taste and aroma, is recognized as one of the world's six major distilled liquors. Among the various types of Chinese Baijiu, the strong-aroma type holds a dominant position. As is well known, the basic components of liquor are consistent, primarily ethanol and water, accounting for approximately 98% of the total components. The remaining 2% consists of chromatographic backbone compounds and trace flavor substances that define the unique flavor style of Chinese Baijiu. To date, more than 470 aroma components have been identified in Chinese Baijiu, mainly including esters, organic acids, alcohols, aldehydes, ketones, and aromatic compounds, most of which originate from microbial fermentation during the brewing process. Although these components are present in trace amounts, they significantly influence the style and characteristics of Chinese Baijiu.

Specifically, ethyl 3-hydroxybutyrate, characterized by fruity, grape-like, and fresh notes together with a Baijiu-like aroma, is an aromatic compound found in wine and *Goeppertia insignis.*

2,3-dihydrobenzofuran, commonly known as coumaran, is a fine chemical raw material widely used in the synthesis of tricyclic compounds and serves as an important intermediate in the synthesis of some pharmaceuticals, including antitumor agents (benzofuran sulfonylurea compounds) and matrix metalloproteinase inhibitors (arylsulfonamide-based carboxylic acids). Therefore, 2,3-dihydrobenzofuran has promising market prospects as a pharmaceutical intermediate. Currently, 2,3-dihydrobenzofuran is primarily prepared through chemical synthesis methods, which can be generally categorized into three types: (1) ortho-disubstituted cyclization on the benzene ring; (2) hydrogenation of benzofuran; and (3) mono-substituted cyclization on the benzene ring. However, these chemical synthesis methods often require a variety of solvents, some of which are toxic and environmentally unfriendly, and typically result in low yields.

4-vinylguaiacol has a strong spicy, clove-like, and fermented aroma with roasted peanut notes. It is a volatile compound with distinctive fermented characteristics and high olfactory recognition. As an important flavor substance in soy sauce, Chinese Baijiu, wine, and beer, it plays an indispensable role in the overall flavor evaluation of these fermented products. In addition, 4-vinylguaiacol is also a highly valuable fragrance compound widely used in the daily chemical, pharmaceutical, and flavor industries, with a market value approximately 40 times higher than that of ferulic acid. It can also be further converted into various high-value-added compounds such as ethyl guaiacol, vanillin, vanillyl alcohol, and vanillic acid. In recent years, the growing popularity of natural products has generated increasing interest in aroma components. 4-vinyl derivatives have been approved by regulatory authorities for use as flavoring agents and meat preservatives. Apart from its value as a fragrance and additive, 4-vinylguaiacol also offers potential pharmaceutical applications in ophthalmic products, such as its applications in ophthalmology as contact lens solutions and eye drops, due to its antioxidant and anticancer activities. In recent years, the global demand for natural aromatic compounds has been gradually increasing. Therefore, it is essential to find safe and efficient microorganisms for the biosynthesis of natural 4-vinylguaiacol.

Currently, functional microbial strains capable of simultaneously producing ethyl 3-hydroxybutyrate, 2,3-dihydrobenzofuran, and 4-vinylguaiacol are few and rarely reported. Therefore, further development of such microbial strains is urgently needed.

### SUMMARY

The present disclosure aims to provide a strain of *Priestia* sp. capable of simultaneously producing ethyl 3-hydroxybutyrate, 2,3-dihydrobenzofuran and 4-vinylguaiacol, so as to solve the problems that scarce functional microorganisms are available for co-producing ethyl 3-hydroxybutyrate, 2,3-dihydrobenzofuran and 4-vinylguaiacol in existing reports, and the same needs to be developed.

To achieve the above objectives, the present disclosure implements the following technical solutions:

In a first aspect, the present disclosure provides *Priestia* sp. capable of producing ethyl 3-hydroxybutyrate, 2,3-dihydrobenzofuran and 4-vinylguaiacol, where the *Priestia* sp. has a deposit number of CGMCC No. 29032. The strain was deposited on November 16, 2023 at China General Microbiological Culture Collection Center (CGMCC), located at No. 3, Yard 1, Beichen West Road, Chaoyang District, Beijing, 100101, China (Institute of Microbiology, Chinese Academy of Sciences). The strain is taxonomically designated as *Priestia* sp.

A nucleotide sequence of the 16S rDNA of the above-mentioned *Priestia* sp. is shown as SEQ ID NO:1.

SEQ ID NO:1, the nucleotide sequence of the 16S rDNA of the *Priestia* sp.

GGGGAATGCTACAGCTAGACATGCAGTCGAGCGAACTGATTAGAAGCTTGCTTCTATGAC
GTTAGCGGCGGACGGGTGAGTAACACGTGGGCAACCTGCCTGTAAGACTGGGATAACTTCGGGAA
ACCGAAGCTAATACCGGATAGGATCTTCTCCTTCATGGGAGATGATTGAAAGATGGTTTCGGCTATC
ACTTACAGATGGGCCCGCGGTGCATTAGCTAGTTGGTGAGGTAACGGCTCACCAAGGCAACGATGC
ATAGCCGACCTGAGAGGGTGArCGGCCACACTGGGACTGAGACACGGCCCAGACTCCTACGGGAG
GCAGCAGTAGGGAArCTTCCGCAATGGACGAAAGTCTGACGGAGCAACGCCGCGTGAGTGATGAA
GGCTTTCGGGTCGTAAAACTCTGTTGTTAGGGAAGAACAAGTACGAGAGTAACTGCTCGTACCTTG
ACGGTACCTAACCAGAAAGCCACGGCTAACTACGTGCCAGCAGCCGCGGTAATACGTAGGTGGCA
AGCGTTATCCGGAATTATTGGGCGTAAAGCGCGCGCAGGCGGTTTCTTAAGTCTGATGTGAAAGCC
CACGGCTCAACCGTGGAGGGTCATTGGAAACTGGGGAACTTGAGTGCAGAAGAGAAAAGCGGAA
TTCCACGTGTAGCGGTGAAATGCGTAGAGATGTGGAGGAACACCAGTGGCGAAGGCGGCTTTTTG
GTCTGTAACTGACGCTGAGGCGCGAAAGCGTGGGGAGCAAACAGGATTAGATACCCTGGTAGTCC
ACGCCGTAAACGATGAGTGCTAAGTGTTAGAGGGTTTCCGCCCTTTAGTGCTGCAGCTAACGCATT
AAGCACTCCGCCTGGGGAGTACGGTCGCAAGACTGAAACTCAAAGGAATTGACGGGGGCCCGCA
CAAGCGGTGGAGCATGTGGTTTAATTCGAAGCAACGCGAAGAACCTTACCAGGTCTTGACATCCTC
TGACAACTCTAGAGATAGAGCGTTCCCCTTCGGGGGACAGAGTGACAGGTGGTGCATGGTTGTCGT
CAGCTCGTGTCGTGAGATGTTGGGTTAAGTCCCGCAACGAGCGCAACCCTTGATCTTAGTTGCCAG
CATTTAGTTGGGCACTCTAAGGTGACTGCCGGTGACAAACCGGAGGAAGGTGGGGATGACGTCA
AACATCATGCCCCTTATGACCTGGGCTACACACGTGCTACAATGGATGGTACAAAGGGCTGCAAGA

CCGCGAGGTCAAGCCAATCCCATAAAACCATTCTCAGTTCGGATTGTAGGCTGCAACTCGCCTACAT
GAAGCTGGAATCGCTAGTAATCGCGGATCAGCATGCCGCGGTGAATACGTTCCCGGGCCTTGTACA
CACCGCCCGTCACACCACGAGAGTTTGTAACACCCGAAGTCGGTGGAGTAACCGTAAGGAGCTAG
CCTACCTATAGAGTTCCCCCCG.

The cellular characteristics of the *Priestia* sp. indicate that it is a spherical bacterium. When cultured on NA medium, the colonies are milky white, opaque, with smooth and moist surfaces and regular edges.

The nutrient agar (NA) medium consists of: 3 parts beef extract, 5 parts peptone, 2.5 parts glucose, dissolved in 1,000 mL distilled water, with pH adjusted to 7.0 using NaOH.

The *Priestia* sp. has the biological characteristics of being capable of utilizing glycerol, L-arabinose, D-ribose, D-galactose, D-glucose, D-fructose, inositol, D-mannitol, methyl-α-D-mannopyranoside, methyl-α-D-glucopyranoside, N-acetylglucosamine, amygdalin, arbutin, aesculin (ferric citrate), salicin, D-cellobiose, D-maltose, D-lactose, D-melibiose, D-sucrose, D-trehalose, inulin, D-melezitose, D-raffinose, starch, glycogen, D-gentiobiose, and D-talose.

The strain is incapable of utilizing erythritol, D-arabinose, D-xylose, L-xylose, D-ribitol, methyl-β-D-xylopyranoside, D-mannose, L-sorbose, L-rhamnose, dulcitol, sorbitol, D-lyxose, D-tagatose, D-fucose, L-fucose, D-arabitol, L-arabitol, potassium gluconate, 2-keto-gluconate, and 5-keto-gluconate.

The *Priestia* sp. produces ethyl 3-hydroxybutyrate at a level of 1.53 ppm.

The *Priestia* sp. produces 2,3-dihydrobenzofuran at a level of 0.38 ppm.

The *Priestia* sp. produces 4-vinylguaiacol at a level of 0.29 ppm.

In a second aspect, the present disclosure provides use of the *Priestia* sp. in the production of ethyl 3-hydroxybutyrate, 2,3-dihydrobenzofuran and 4-vinylguaiacol.

In a third aspect, the present disclosure provides use of the *Priestia* sp. in the brewing of a distilled liquor or a fermented liquor.

Specifically, the distilled liquor is at least one selected from Chinese Baijiu, brandy, whisky, vodka, rum, gin, tequila, or fruit-distilled liquor.

The fermented liquor is at least one selected from Huangjiu, beer, grape wine, fruit wine, sake, or milk wine.

Preferably, the Chinese Baijiu includes at least one of strong-aroma type, sauce-aroma type, light-aroma type, or mixed-aroma type.

In a fourth aspect, the present disclosure provides use of the *Priestia* sp. in the preparation of fermented foods, flavorings, additives, or pharmaceuticals.

In a fifth aspect, the present disclosure provides a fermentation broth, seed liquid, or bacterial cells containing the *Priestia* sp.

In a sixth aspect, the present disclosure provides a method for isolating and screening the *Priestia* sp., including the following steps:
collecting samples from a brewing environment of a distillery workshop, culturing on a PCA medium, selecting colonies and streaking to obtain the desired strain.

Specifically, the PCA medium consists of: 5 g/L tryptone, 2.5 g/L yeast extract powder, 1 g/L glucose, and 2 g/L natamycin.

In a seventh aspect, the present disclosure provides a method for the production of ethyl 3-hydroxybutyrate, 2,3-dihydrobenzofuran and 4-vinylguaiacol using the *Priestia* sp., including the following steps: inoculating the *Priestia* sp. into a sorghum culture medium and then fermenting to obtain the target product.

Specifically, an inoculation amount of the strain is 5%, a culture temperature is 32°C, and a culture duration is 2 d.

The sorghum culture medium is prepared by gelatinizing sorghum with water, liquefying with α-amylase, saccharifying with glucoamylase, and adjusting a sugar concentration.

Further, the sorghum culture medium is prepared by adding 200 g of sorghum to 3 L of water and gelatinized for 40 min., 5 g of α-amylase is then added for liquefaction at 65°C for 3 h, followed by 5 g of glucoamylase at 62°C for 3 h, a sugar concentration is adjusted to 12°Bé.

Beneficial effects: a strain of *Priestia* sp. of the present disclosure was isolated from a strong-aroma Baijiu brewing environment of WULIANGYE YIBIN CO., LTD, and designated as WMCC10082, with a deposit number of CGMCC NO.29032. The strain is capable of simultaneously producing ethyl 3-hydroxybutyrate, 2,3-dihydrobenzofuran, and 4-vinylguaiacol. Therefore, the *Priestia* sp. has broad application prospects in the fields of winemaking, fermented foods, fragrances and food additives using ethyl 3-hydroxybutyrate, 2,3-dihydrobenzofuran and 4-vinylguaiacol for flavoring, and also in the pharmaceutical field utilizing the antioxidant activity and the anticancer activity of 4-vinylguaiacol.

### BRIEF DESCRIPTION OF THE DRAWINGS

FIG. 1 illustrates colony morphology of *Priestia* sp. (CGMCC NO. 29032) on an NA medium (left) and cells observed under a microscope after Gram staining (right) according to the present disclosure.
FIG. 2 illustrates a phylogenetic tree based on a 16S rDNA sequence of *Priestia* sp. (CGMCC NO. 29032) according to the present disclosure.
FIG. 3 illustrates an HS-SPME chromatogram of a fermentation broth of *Priestia* sp. (CGMCC NO. 29032) according to the present disclosure.
FIG. 4 illustrates an HS-SPME chromatogram of a blank fermentation broth.

The present disclosure provides *Priestia* sp. capable of producing ethyl 3-hydroxybutyrate, 2,3-dihydrobenzofuran and 4-vinylguaiacol, where the *Priestia* sp. has a deposit number of CGMCC No. 29032. The strain was deposited on November 16, 2023 at China General Microbiological Culture Collection Center (CGMCC), located at No. 3, Yard 1, Beichen West Road, Chaoyang District, Beijing, 100101, China (Institute of Microbiology, Chinese Academy of Sciences). The strain is taxonomically designated as *Priestia* sp.

### DETAILED DESCRIPTIONS OF THE EMBODIMENTS

In order to make the technical problems, technical solutions, and beneficial effects of the present disclosure clearer and more understandable, the present disclosure is described in detail below in conjunction with specific embodiments. Unless otherwise defined, all technical terms used herein have the same meanings as commonly understood by those skilled in the art.

The present disclosure provides a strain of *Priestia* sp., which was isolated from a strong-aroma Baijiu brewing environment of Yibin Wuliangye Co., Ltd., and designated as WMCC10082. The strain of the present disclosure was deposited on November 16, 2023, with a deposit number of CGMCC No. 29032, at China General Microbiological Culture Collection Center (CGMCC), located at No. 3, Yard 1, Beichen West Road, Chaoyang District, Beijing, 100101, China (Institute of Microbiology, Chinese Academy of Sciences).

*Priestia* sp. WMCC10082 of the present disclosure is capable of simultaneously producing ethyl 3-hydroxybutyrate, 2,3-dihydrobenzofuran, and 4-vinylguaiacol.

The following embodiments are provided to illustrate the embodiments of the present disclosure. Those skilled in the art will understand that the following embodiments are only used to illustrate the present disclosure, and should not be considered as limiting the scope of the present disclosure. Where specific techniques or conditions are not specified in the examples, they shall be performed according to the techniques or conditions described in the literature in the art or according to the product instructions. Reagents or instruments used without indicating manufacturers are conventional products commercially available.

The plate count agar (PCA) medium for isolation and culture medium used in the examples consists of: 5 parts tryptone, 2.5 parts yeast extract powder, 1 part glucose, 2 parts natamycin, dissolved in 1000 parts distilled water. After mixing, a pH is adjusted to 7.2 with NaOH.

The nutrient agar (NA) medium used in the examples consists of: 3 parts beef extract, 5 parts peptone, 2.5 parts glucose, dissolved in 1000 mL distilled water, with pH adjusted to 7.0 using NaOH.

Slants and plates in the examples were prepared by adding 2% agar powder to the corresponding medium.

### Example 1 Isolation, Screening, and Identification of Priestia sp. WMCC10082

### (I) Isolation and Screening

Samples collected from a brewing environment of Yibin Wuliangye Co., Ltd. were plated on the PCA medium for bacterial screening. Air samples were collected for 1 min (100 L of air) using a planktonic bacteria sampler (Model FKC-I, Zhejiang Sujing Purification Equipment Co., Ltd.), with sampling repeated three times. The samples were statically incubated at 37 °C for 3 d. Colonies on the plates were selected for streak culture, repeated three times until purified strains were obtained, which were then preserved on slant culture. The strain obtained was designated WMCC10082.

### (II) Classification and Identification

### 1. Morphological characteristics of the strain

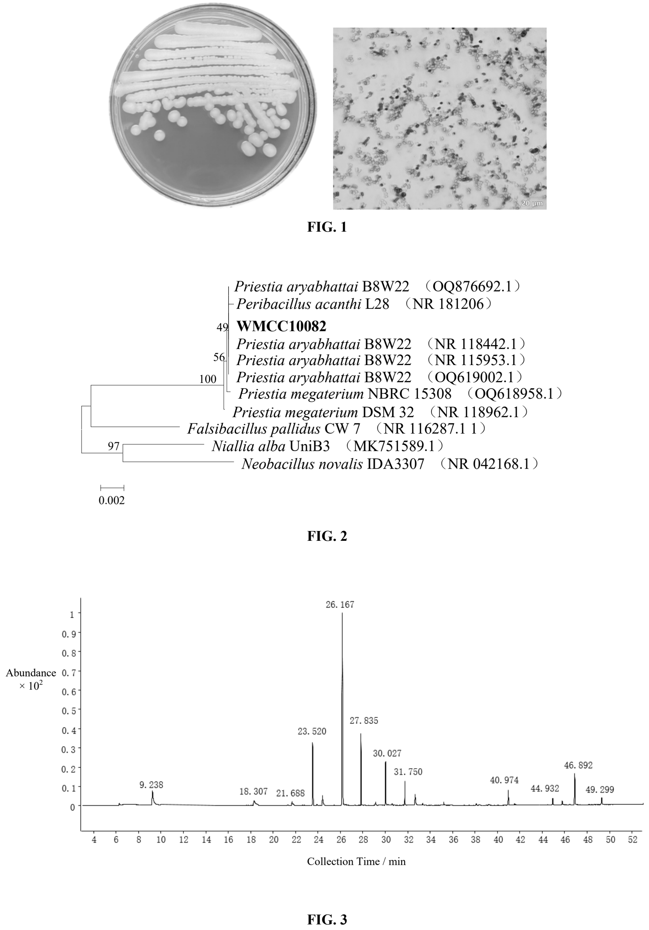

The isolated strain WMCC10082 was a Gram-negative, spherical bacterium. When cultured on NA medium, the colonies were milky white, opaque, having smooth and moist surfaces and regular edges, as shown in FIG. 1.

### 2. Physiological and biochemical characteristics

The strain WMCC10082 was inoculated onto the NA plates and statically incubated at 37 °C for 3 d. Identification was performed using the API 50CH physiological and biochemical identification kit. Results showed that the strain was capable of utilizing glycerol, L-arabinose, D-ribose, D-galactose, D-glucose, D-fructose, inositol, D-mannitol, methyl-α-D-mannopyranoside, methyl-α-D-glucopyranoside, N-acetylglucosamine, amygdalin, arbutin, aesculin (ferric citrate), salicin, D-cellobiose, D-maltose, D-lactose, D-melibiose, D-sucrose, D-trehalose, inulin, D-melezitose, D-raffinose, starch, glycogen, D-gentiobiose, and D-talose.

The strain was incapable of utilizing erythritol, D-arabinose, D-xylose, L-xylose, D-ribitol, methyl-β-D-xylopyranoside, D-mannose, L-sorbose, L-rhamnose, dulcitol, sorbitol, D-lyxose, D-tagatose, D-fucose, L-fucose, D-arabitol, L-arabitol, potassium gluconate, 2-keto-gluconate, and 5-keto-gluconate.

### 3. 16S rDNA Identification

The strain WMCC10082 was inoculated into NA medium and incubated at 37 °C for 3 d. 1 mL of the bacterial suspension was collected and centrifuged to harvest a bacterial cell pellet. Genomic DNA was extracted using a bacterial genomic DNA extraction kit, and the DNA was then stored at -20°C for subsequent use.

Bacterial genomic 16S rDNA was amplified using primers 27F and 1492R with sequences as follows: 27F: AGAGTTTGATCMTGGCTCAG(SEQ ID NO:2), 1492R: GGTTACCTTGTTACGACTT(SEQ ID NO:3).

An amplification system (25 µL in total) consisted of: 2.5 µL of 10×PCR buffer, 1µL of dNTP (2.5mmol/L each), 10 ng of DNA, 0.5 µL of primer F (10 µmol/L), 0.5 µL of primer R (10 µmol/L), and double distilled water added to a final volume of 25 µL. Amplification procedures were as follows: 94°C for 4 min; followed by 30 cycles of 94°C for 45 s, 55°C for 45 s, and 72°C for 60 s; final extension at 72°C for 10 min; and reaction terminated at 4°C.

16S rDNA sequence determination: PCR amplification products were purified using a gel extraction kit. Sequencing of the purified products was performed by China Center of Industrial Culture Collection (CICC). The 16S rDNA sequence of WMCC10082 is shown as SEQ ID NO: 1.

Phylogenetic tree construction: The obtained bacterial 16S rDNA gene sequences were analyzed for similarity using BLASTn against the GenBank database. Standard strains with relatively close phylogenetic relationships were selected, and sequence alignment was performed using ClustalW software. Phylogenetic analysis was performed using MEGA7 software, and a phylogenetic tree was constructed, as shown in FIG. 2.

Based on comprehensive analysis of cell morphology, physiological and biochemical characteristics, and 16S rDNA sequence, WMCC10082 was identified as *Priestia* sp. WMCC10082 was deposited on November 16, 2023, with a deposit number of CGMCC No. 29032, at China General Microbiological Culture Collection Center (CGMCC), located at No. 3, Yard 1, Beichen West Road, Chaoyang District, Beijing, 100101, China (Institute of Microbiology, Chinese Academy of Sciences).

### Example 2: Determination of Flavor Compounds Produced by Priestia sp. WMCC10082

1. Preparation of a sorghum culture medium: 200 g of sorghum was added to 3 L of water and gelatinized for 40 min., 5 g of α-amylase was then added for liquefaction at 65°C for 3 h, followed by 5 g of glucoamylase at 62°C for 3 h, a sugar concentration was adjusted to 12 °Bé, and a resulting mixture was filtered through gauze, sterilized, and reserved for use.

The above-mentioned strain was inoculated into the prepared sorghum culture medium at a 5% inoculation amount and cultured at 32°C with shaking at 120 rpm for 2 d. A resulting fermentation broth was then collected for later use.

2. Detection method: The fermentation broth was centrifuged at 5,000 r/min for 10 min, and 2 mL of the supernatant was filtered through a 0.22 µm filter membrane. Volatile flavor compounds thereof were determined using headspace solid-phase microextraction coupled with gas chromatography-mass spectrometry (HS-SPME-GC-MS).
(1) HS-SPME conditions: 2 mL of the filtered fermentation broth was placed in a 20 mL headspace vial, and 10 µL of internal standard (4-octanol) was added. The fermentation broth was equilibrated at 60°C for 15 min. An SPME needle was inserted at a position approximately 1 cm above a fermentation broth surface, adsorption was performed for 40 min, and desorption in an injection port was performed for 3 min.
(2) GC conditions: a TG-WAXMS column (30.0 m × 0.25 mm × 0.25 µm) was used, a temperature in the injection port was set at 250°C; Helium (He) was used as a carrier gas at a flow rate of 1 mL/min in splitless injection mode. Programmed temperature gas chromatography was as follows: an initial temperature was 40°C, held for 5 min, then increased at 4 °C/min to 230°C, held for 15 min.
(3) MS conditions: The mass spectrometer was operated in electron ionization (EI) mode at 70 eV, an ion source temperature was set at 200°C, and a quadrupole temperature was set at 150°C. A mass spectrum scanning range was 35-350 amu.

4-octanol (1.0 ppm) was used as an internal standard, and a semi-quantitative analysis was performed using a peak area normalization method. A peak area of each compound was calculated using selected ion monitoring (SIM) method, and a relative concentration of each compound was calculated according to a ratio of its peak area to that of the internal standard.

3. Results: In the sorghum culture medium, the main flavor compounds produced by *Priestia* sp. WMCC10082 were ethyl 3-hydroxybutyrate, 2,3-dihydrobenzofuran, and 4-vinylguaiacol.
(1) HS-SPME chromatogram

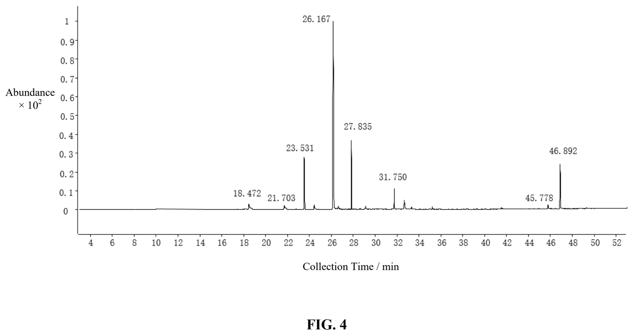

The chromatogram of the WMCC10082 fermentation broth is shown in FIG. 3, and the chromatogram of a blank fermentation broth is shown in FIG. 4.

(2) Flavor compound information is shown in Table 1.

**Table 1 Information of flavor compounds produced by Priestia sp. WMCC10082**

| **Name** | **Retention time** | **CAS No.** | **Blank (ppm)** | **WMCC10082 (ppm)** |
|---|---|---|---|---|
| Ethyl 3-hydroxybutyrate | 30.027 | 5405-41-4 | 0.00 | 1.53 |
| 4-vinylguaiacol | 44.932 | 7786-61-0 | 0.00 | 0.29 |
| 2,3-dihydrobenzofuran | 49.299 | 496-16-2 | 0.00 | 0.38 |

As shown in Table 1, the main flavor compounds produced by *Priestia* sp. WMCC10082 are ethyl 3-hydroxybutyrate, 2,3-dihydrobenzofuran, and 4-vinylguaiacol, with concentrations of 1.53 ppm, 0.38 ppm and 0.29 ppm, respectively, all of which are important flavor compounds in Chinese Baijiu. The strain of the present disclosure can be widely applied in the brewing of strong-aroma Baijiu to improve the flavor profile of Chinese Baijiu, providing a novel microorganism for aroma enhancement of strong-aroma Baijiu and offering broad application prospects.

## Claims

1. *Priestia* sp. capable of producing ethyl 3-hydroxybutyrate, 2,3-dihydrobenzofuran and 4-vinylguaiacol, wherein the *Priestia* sp. has a deposit number of CGMCC No. 29032.

2. Use of the *Priestia* sp. according to claim 1 in the production of ethyl 3-hydroxybutyrate, 2,3-dihydrobenzofuran and 4-vinylguaiacol.

3. Use of the *Priestia* sp. according to claim 1 in the brewing of a distilled liquor or a fermented liquor.

4. The use according to claim 3, wherein the distilled liquor is at least one selected from Chinese Baijiu, brandy, whisky, vodka, rum, gin, tequila, or fruit-distilled liquor, and the fermented liquor is at least one selected from Huangjiu, beer, grape wine, fruit wine, sake, or milk wine.

5. The use according to claim 4, wherein the Chinese Baijiu comprises at least one of strong-aroma type, sauce-aroma type, light-aroma type, or mixed-aroma type.

6. Use of the *Priestia* sp. according to claim 1 in the preparation of fermented foods, flavorings, additives, or pharmaceuticals.

7. A microbial agent, comprising a fermentation broth, seed liquid, or bacterial cells containing the *Priestia* sp. according to claim 1.

8. A method for isolating and screening the *Priestia* sp. according to claim 1, comprising the following steps: collecting samples from a brewing environment of a distillery workshop, culturing the samples on a plate count agar (PCA) medium, and selecting colonies for streak culture to obtain the *Priestia* sp., wherein the PCA medium comprises 5 g/L tryptone, 2.5 g/L yeast extract powder, 1 g/L glucose, and 2 g/L natamycin.

9. A method for producing ethyl 3-hydroxybutyrate, 2,3-dihydrobenzofuran and 4-vinylguaiacol using the *Priestia* sp. according to claim 1, comprising the following steps: inoculating the *Priestia* sp. into a sorghum culture medium, and then fermenting the sorghum culture medium to obtain the ethyl 3-hydroxybutyrate, the 2,3-dihydrobenzofuran and the 4-vinylguaiacol.

10. The method according to claim 9, wherein at least one of the following conditions is satisfied:
an inoculation amount of the strain is 5%, a culture temperature is 32°C, and a culture duration is 2 d; or
the sorghum culture medium is prepared by gelatinizing sorghum with water, liquefying with α-amylase, saccharifying with glucoamylase, and adjusting a sugar concentration; preferably, the sorghum culture medium is obtained by adding 200 g of sorghum to 3 L of water, gelatinizing for 40 min, adding 5 g of α-amylase and liquefying at 65°C for 3 h, adding 5 g of glucoamylase and saccharifying at 62°C for 3 h, and adjusting the sugar concentration to 12 °Bé.
